(19) Europäisches Patentamt / European Patent Office / Office européen des brevets

(11) **EP 2 386 628 A1**

(12) **EUROPEAN PATENT APPLICATION**

(43) Date of publication:
**16.11.2011 Bulletin 2011/46**

(51) Int Cl.:
***C12N 7/02*** (2006.01)

(21) Application number: **11167550.0**

(22) Date of filing: **25.05.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.04.2010 US 325954 P**

(71) Applicant: **Millipore Corporation**
**Billerica, MA 01821 (US)**

(72) Inventors:
- **Ramaswamy, Senthil**
  **Billerica, MA 01821 (US)**
- **Cross, Steve**
  **Billerica, MA 01821 (US)**
- **Iyer, Ganesh**
  **Billerica, MA 01821 (US)**

(74) Representative: **UEXKÜLL & STOLBERG**
**Patentanwälte**
**Beselerstraße 4**
**22607 Hamburg (DE)**

(54) **Separation of viruses from nucleic acids using a membrane coated with polymeric primary amines**

(57) A method of purifying biomolecules with an anion exchanger containing a membrane having a surface having a polymer such as a primary or secondary amine ligand formed thereon, such as polyallylamine. The feedstock is introduced to the exchanger in the presence of one or more ionic-modifiers by themselves or in combination with monovalent salt. The ionic modifier alters the binding ability of the primary amines such that they retain a significant binding capacity for highly charged species such as DNA but lose part or almost all of their binding capacity for less charged species such as viruses or proteins at pH above the pI of the virus or protein.

EP 2 386 628 A1

**Description**

This application claims priority of U.S. Provisional Application No. 61/325,954 filed April 20, 2010, the disclosure of which is incorporated herein by reference.

BACKGROUND OF THE INVENTION

The embodiments disclosed herein relate to separation of viruses and/or protein from nucleic acids with primary amines.

Strong anion exchangers, such as those based on quarternary ammonium ions, are used in, for example, downstream processing as a polishing media for capturing negatively charged large impurities, such as endotoxins, viruses, nucleic acids, and host cell proteins (HCP) that are present in fluids such as biological fluids, particularly solutions of manufactured biotherapeutics. Traditionally, anion exchangers have been offered and used in the bead format, for example Q Sepharose® available from GE Healthcare Bio-Sciences AB. However, throughput limitations of bead-based systems can require large volume columns to effectively capture impurities.

Membrane-based chromatographic systems (also called membrane sorbers), have the ligands attached directly to the convective membrane pores, thereby eliminating the effects of internal pore diffusion on mass transport. Additionally, the use of microporous membrane substrates with a tight membrane pore size distribution coupled with effective flow distributors can minimize axial dispersion and provide uniform utilization of all active sites. Consequently, mass transfer rates of membrane sorber media may be an order of magnitude greater than that of standard bead-based chromatography media, allowing for both high efficiency and high-flux separations. Since single or even stacked membranes are very thin compared to columns packed with bead-based media, reduced pressure drops are found along the chromatographic bed, thus allowing increased flow rates and productivities. The necessary binding capacity is reached by using membranes of sufficient internal surface area, yielding device configurations of very large diameter to height ratios (d/h).

Properly designed membrane sorbers have chromatographic efficiencies that are 10 - 100 times better than standard preparative bead-based resins. Consequently, to achieve the same level of separation on a membrane sorber, a bed height 10-fold less can be utilized. Bed lengths of 1-5 mm are standard for membrane sorbers, compared to bed heights of 10-30 cm for bead-based systems. Due to the extreme column aspect ratios required for large-volume membrane sorbers, device design is critical. To maintain the inherent performance advantages associated with membrane sorbers, proper inlet and outlet distributors are required to efficiently and effectively utilize the available membrane volume. Membrane sorber technology is ideally suited for this application.

A membrane sorber is a highly porous, interconnected media that has the ability to remove (ad- and/or absorb) some components of a solution when the latter flows through its pores. The properties of the membrane sorber and its ability to perform well in the required application depend on the porous structure of the media (skeleton) as well as on the nature of the surface that is exposed to the solution. Typically, the porous media is formed first, from a polymer that does not dissolve or swell in water and possesses acceptable mechanical properties. The porous media is preferably a porous membrane sheet made by phase separation methods well known in the art. See, for example, Zeman LJ, Zydney AL, Microfiltration and Ultrafiltration: Principles and Applications, New York: Marcel Dekker, 1996. Hollow fiber and tubular membranes are also acceptable skeletons. A separate processing step is usually required to modify the external or facial surfaces and the internal pore surfaces of the formed porous structure to impart the necessary adsorptive properties. Since the membrane structure is often formed from a hydrophobic polymer, another purpose of the surface modification step is also to make the surfaces hydrophilic, or water-wettable.

There exist a number of approaches to modify the external or facial surfaces and the internal pore surfaces of a membrane. Those skilled in the art will readily recognize exemplary methods involving adsorption, plasma oxidation, in-situ free-radical polymerization, grafting and coating. The majority of these methods lead to formation of monolayer-like structures on the membrane surface, which most of the time achieve the goal of making it hydrophilic, yet fail to impart acceptable adsorptive properties, for example high capacity for the adsorbate. The capacity is defined as the amount (weight) of the adsorbate that can be retained by a given volume of the media. As long as all adsorption occurs on the membrane surface, the capacity will be limited by the membrane surface area. By their nature, microporous membranes have lower surface area compared to chromatography beads. One way to increase surface area is to reduce pore size, which obviously leads to significant losses in flux. For example, the maximum (monolayer) adsorption of protein on a 0.65 um polyethylene membrane (Entegris Corp, Billerica MA) is about 20 mg/ml, regardless of the type of surface interactions. This is significantly less than, for example, agarose chromatography beads, with typical capacity about 80 mg/ml.

The type of surface interactions driving the adsorption is defined by the specific application in which a given membrane sorber product is used. For example, co-pending Application Serial No. 12/221,496 discloses a high-capacity, high-affinity sorber that removes viruses, nucleic acids, endotoxins, and host cell proteins (HCPs) from a solution of monoclonal antibodies (MABs). These impurities tend to have a lower isoelectric point than the MABs, which means that at a certain pH they will be negatively charged while the MAB will be positively charged. An anion exchanger, i.e. a media that bears a positive charge and attracts anions, is required to remove these impurities. A number of chemical moieties bear a positive charge in an aqueous solution, including primary, secondary, and tertiary amines, as well as quaternary ammonium salts. The amines are positively charged at pH below 11, while the ammonium salts bear the positive charge at all pH, so these groups are commonly called weak and strong anion exchangers, respectively.

Anion exchange membranes have multiple positively charged binding sites that attract and hold various impurities and contaminants. The amount of impurities that can be potentially removed is a function of the concentration of these binding sites on the membrane, and the chemical nature of the ligand (as well as the concentration of these ligands) is responsible for the strength of binding for the various impurities. High strength of binding is a key attribute for increasing the removal of impurities, for example, host cell proteins. Strength of binding (SB) is also related to the ionic strength of solutions required to elute the bound impurities. SB of a membrane sorber (measured in conductivity units, mS/cm) is determined as follows. First, a small amount of adsorbate solution is passed through the membrane sorber so the adsorbate binds to the membrane sorber. Second, the membrane sorber is eluted with increasing gradient of inorganic salt, such as sodium chloride. The minimum conductivity of elution solution required to elute off the adsorbate is recorded and defined as the SB of that membrane sorber. By increasing the sorber strength of binding, negatively charged impurities can be made to bind irreversibly to the membrane sorber, thereby significantly increasing the removal efficiency. The high strength of binding translates to the ability to bind negatively charged impurities such as viruses or DNA at very high salt concentrations.

In the vaccine industry, it is essential that viruses or proteins used in the vaccines be as pure as possible, and thus it is desirable to purify viruses from impurities such as nucleic acids, particularly DNA. In order to carry out such a purification using chromatography in flow-through mode, it is necessary that the DNA bind to the membrane while the viruses/proteins flow through the membrane. In order to effectuate this using Anion exchange chromatography, normally the pH of the feed must be below the pI of the virus or protein (often about 5.5 or less) which could lead to instability of these bimolecules and inactivation.

Accordingly, it would be desirable to provide a method for effectively and selectively separating viruses or proteins from, for example, DNA with media using a flow-through anion exchanger including such media.

## SUMMARY OF THE INVENTION

The problems of the prior art have been overcome by the present invention, which provides for the separation of biomolecules such as viruses and other proteins from nucleic acids, particularly host cell DNA, using anion exchangers including a membrane having a surface having a polymer such as a primary or secondary amine ligand formed thereon, such as polyallylamine. Described is a method of modulating the binding capacity of microporous membranes so that they selectively retain a significant and effective bonding capacity for highly charges species such as DNA while losing substantially all their binding capacity for charged species such as viruses or proteins at pH above the pI of the virus or proteins. The result is the effective separation of biomolecules such as viruses/proteins from DNA by establishing conditions such that the biomolecule flows through the device at a pH above the pI of the biomolecule while still retaining significant DNA binding.

In certain embodiments, the entire external and internal pore surfaces are formed with a loosely cross-linked hydrogel. The wet (swollen) thickness of this hydrogel is about 50-100 nm. The binding capacity of polymeric primary amines, and secondary amines, preferably aliphatic polymers having a primary amine covalently attached to the polymer backbone, more preferably having a primary amine covalently attached to the polymer backbone by at least one aliphatic group, preferably a methylene group, is modulated with buffers containing or composed of one or more ionic-modifiers by themselves or in combination with monovalent salts.

An ionic modifier is defined as a molecule that has more than one ionizable group and which can strongly interact with the primary amine groups on the membrane and/or strongly interact with the biomolecule preventing it from binding to the membrane surface and/or which can from weak or strong bridges between two or more similarly charged ionic groups or molecules. These molecules include but are not limited to multivalent ions or salts such as phosphates, sulfates or borates and polyionic molecules such as citrates, ethylenediaminetetraacetic acid, polyacrylic acid etc. These molecules may be used in their acid, base or salt form. Monovalent salts are salts which have a single ionization, such as sodium chloride, sodium bromide, sodium iodide etc.

## BRIEF DESCRIPTION OF THE DRAWINGS

Figure 1 is a graph of Flu-A titer and DNA concentration in the flow through stream of an adsorber in accordance with certain embodiments;

Figure 2 is a graph of Flu-A titer concentration in the flow through stream of an adsorber in accordance with certain embodiments; and

Figure 3 is a graph of DNA concentration in the flow through stream of an adsorber in accordance with certain embodiments.

## DETAILED DESCRIPTION OF SPECIFIC EMBODIMENTS

The embodiments disclosed herein relate to methods of purification involving a porous chromatographic or sorptive media having a porous polymer formed on a porous, self-supporting substrate. The media is particularly suited for the robust removal of species such as DNA from viruses in the presence of one or more ionic-modifiers such as phosphate, citrate, ethylenediaminetetraacetic acid (EDTA), succinate, ammonium sulfate, sodium sulfate, piperazine-N,N'-bis(2-ethanesulfonic acid), glutamate, tri-polyphosphates, polyelectrolytes such as polyacrylic acid, sodium styrene sulfonate, surfactants such as sodium lauryl ether sulfate at various concentrations. (used either as by themselves or as additives in other buffers).

The porous substrate has two surfaces associated with the geometric or physical structure of the substrate. A sheet will have a top and bottom surface, or a first and a second surface. These are commonly termed "sides." In use, fluid will flow from one side (surface) through the substrate to and through the other side (surface). For hollow fibers and tubular membranes, there is an inside and an outside surface. Flow proceeds from the inside to the outside, or vice versa, depending on design and use.

The thickness dimension between the two surfaces is porous. This porous region has a surface area associated with the pores. In order to prevent confusion related to the terms "surface", "surfaces", or "surface area," or similar usages, the inventors will refer to the geometric surfaces as external or facial surfaces or as sides. The surface area associated with the pores will be referred to as internal or porous surface area.

Porous material comprises the pores, which are empty space, and the solid matrix or skeleton which makes up the physical embodiment of the material. For example, in a non-woven web, the randomly oriented fibers make up the matrix and give the web its form. In polymer microporous membranes, the phase separated polymer provides the matrix. Herein, the inventors discuss coating or covering the surface of the media. The inventors mean by this that the internal and external surfaces are coated so as to not completely block the pores, that is, to retain a significant proportion of the structure for convective flow. In particular, for the internal surface area, coating or covering means that the matrix is coated or covered, leaving a significant proportion of the pores open.

Absorption refers to taking up of matter by permeation into the body of an absorptive material. Adsorption refers to movement of molecules from a bulk phase onto the surface of an adsorptive media. Sorption is a general term that includes both adsorption and absorption. Similarly, a sorptive material or sorption device herein denoted as a sorber, refers to a material or device that both ad- and absorbs.

The porous substrate acts as a supporting skeleton for the adsorptive hydrogel. The substrate should be amenable to handling and manufacturing into a robust and integral device. The pore structure should provide for uniform flow distribution, high flux, and high surface area. The substrate may be a fiber, a sheet such as a woven fabric, a non-woven, a mat, a felt or a membrane, or one or more beads. Preferably, the substrate is a sheet formed of a woven or non-woven fabric or a membrane.

Fibers may be of any length, diameter and may be hollow or solid. They are not bonded together as a substrate (although they may be formed into a unitary structure after application of the coating) but are individual discrete entities. They may be in the form of a continuous length such as thread or monofilament of indeterminate length or they may be formed into shorter individual fibers made by chopping fibrous materials such as non-woven or woven fabrics, cutting the continuous length fiber into individual pieces, formed by a crystalline growth method and the like.

Non-woven fabrics are flat, porous sheets made directly from separate fibers bonded together by entangling fiber or filaments, thermally or chemically. Typically, nonwoven fabric manufacturers supply media having from 1 to 500 micron mean flow pore (MFP) ratings. For non-woven fabrics, the porous structure is the entangled fibers, and porosity refers to the tortuous spaces between and among the fibers. Porosity has a similar meaning for felted fabrics. A preferred non-woven is by Freudenberg Nonwovens NA of Lowell, Mass. and is type FO2463.

Woven fabrics are produced by the interlacing of warp fibers and weft fibers in a regular pattern or weave style that is at some predefined angle to each other. Typically the weft is at an angle of about 90 degrees to that of the warp. Other commonly used angles include but are not limited to 30, 45, 60 and 75 degrees. The fabric's integrity is maintained by the mechanical interlocking of the fibers cause by the weaving process. Drape (the ability of a fabric to conform to a complex surface), surface smoothness and stability of a fabric are controlled primarily by the weave style, such as plain, twill, satin, basket weave, leno, etc. In this case, the substrate porosity is the space between the fibers and is of a less tortuous nature.

The substrate also may be formed from a variety of materials including glass, plastics, ceramics and metals.

Borosilicate glass is one example of a suitable glass. It can be formed as fibers or glass mats.

Various ceramics based on the more conventional silicate chemistries or more exotic chemistries such as yttrium, zirconia, titanium and the like and blends thereof can be used. They can be formed into fibers, mats, felts, monoliths or membranes.

Metals such as stainless steel, nickel, copper, iron or other magnetic metals and alloys, palladium, tungsten, platinum, and the like maybe made into various forms including fibers, sintered sheets and structures, such as sintered stainless steel or nickel filters, woven screens and non-woven mats, fabrics and felts such as stainless steel wool.

The preferred substrate is made from synthetic or natural polymeric materials. Thermoplastics are a useful class of polymers for this use. Thermoplastics include but are not limited to polyolefins such as polyethylenes, including ultrahigh molecular weight polyethylenes, polypropylenes, sheathed polyethylene/polypropylene fibers, PVDF, polysulfone, polyethersulfones, polyarylsulphones, polyphenylsulfones, polyvinyl chlorides, polyesters such as polyethylene terephthalate, polybutylene terephthalate and the like, polyamides, acrylates such as polymethylmethacrylate, styrenic polymers and mixtures of the above. Other synthetic materials include celluloses, epoxies, urethanes and the like.

Suitable substrates include microporous filtration membranes, i.e. those with pore sizes from about 0.1 to about 10 $\mu$m. Substrate material can be hydrophilic or hydrophobic. Examples of hydrophilic substrate materials include, but are not limited to, polysaccharides and polyamides, as well as surface treated hydrophilic porous membranes, such as Durapore® (Millipore Corporation, Billerica MA). Examples of hydrophobic material include, but are not limited to, polyolefins, polyvinylidene fluoride, polytetafluoroethylene, polysulfones, polycarbonates, polyesters, polyacrylates, and polymethacrylates. The porous structure is created from the substrate material by any method known to those skilled in the art, such as solution phase inversion, temperature-induced phase separation, air casting, track-etching, stretching, sintering, laser drilling, etc. Because of the universal nature of the present invention, virtually any available method to create a porous structure is suitable for making the supporting skeleton for the membrane sorber. A substrate material made from ultra-high molecular weight polyethylene has been found to be useful due to its combination of mechanical properties, chemical, caustic and gamma stability.

The polymer forms the adsorptive hydrogel and bears the chemical groups (binding groups) responsible for attracting and holding the entities desired to be captured. Alternatively, the polymer possesses chemical groups that are easily modifiable to incorporate the binding groups. The coating or covering is permeable so that impurities can be captured into the depth of the coating or covering, increasing adsorptive capacity. The preferred polymer is a polymeric primary amine. Examples of suitable polymeric primary amines include polyallylamine, polyvinylamine, polybutylamine, polylysine, their copolymers with one another and with other polymers, as well as their respective protonated forms. Suitable copolymers include vinyl alcohol-co-vinylamine, acrylamide-co-allyamine, ethyleneglycol-co-allylamine, and allylamine-co-N-isopropylacrylamide. A coating or covering made from polyallylamine (and/or its protonated form, for example polyallylamine hydrochloride (PAH)) has been found to be particularly useful. PAA is commercially available (Nitto Boseki) in a number of molecular weights, usually in the range from 1,000 to 150,000, and all these can be used for creating a membrane sorber. PAA and PAH are readily soluble in water. The pH of aqueous solution of PAA is about 10-12, while that of PAH is 3-5. PAA and PAH may be used interchangeably, however the pH of the final solution must be monitored and if necessary adjusted to the value above 10 so that non-protonated amino groups are available for reaction with a cross-linker.

The coating or covering typically constitutes at least about 3% of the total volume of the coated or covered substrate, preferably from about 5% to about 10%, of the total volume of the substrate. In certain embodiments, the coating or covering covers the substrate in a substantially uniform thickness. Suitable thicknesses range of dry coating from about 10 nm to about 50 nm.

Those skilled in the art will appreciate that the polymer can be immobilized on the substrate by any suitable means, such as coating, grafting, crosslinking, etc., and the term "coated substrate" as used herein is meant in a broad sense to encompass any substrate having the polymer formed thereon.

A cross-linker can be reacted with the polymer to make the latter insoluble in water and thus held on the surface of the supporting skeleton. Suitable cross-linkers are difunctional or polyfunctional molecules that react with the coating polymer and are soluble in the chosen solvent, which is preferably water. A wide variety of chemical moieties react with primary amines, most notably epoxides, chloro-, bromo-, and iodoalkanes, carboxylic acid anhydrides and halides, aldehydes, $\alpha,\beta$-unsaturated esters, nitriles, amides, and ketones. A preferred cross-linker is polyethylene glycol diglycidyl ether (PEG-DGE). It is readily soluble in water, provides fast and efficient cross-linking, and is hydrophilic, neutral, non-toxic and readily available. The amount of cross-linker used in the coating solution is based on the molar ratio of reactive groups on the polymer and on the cross-linker. The preferred ratio is in the range from about 10 to about 1,000, more preferred from about 20 to about 200, most preferred from about 30 to about 100. More cross-linker will hinder the ability of the hydrogel to swell and will thus reduce the sorptive capacity, while less cross-linker may result in incomplete cross-linking, i.e. leave some polymer molecules fully soluble.

A surfactant may be used to help spread the polymer solution uniformly on the entire surface of the supporting structure. Preferred surfactants are non-ionic, watersoluble, and alkaline stable. Fluorosurfactants possess a remarkable ability to lower water surface tension. These surfactants are sold under the trade name Zonyl by E.I. du Pont de Nemours and Company and are particularly suitable, such as Zonyl FSN and Zonyl FSH. Another acceptable class of surfactants are octylphenol ethoxylates, sold under the trade name Triton X by The Dow Chemical Company. Those skilled in the art will appreciate that other surfactants also may be used. The concentration of surfactant used in the coating solution is usually the minimum amount needed to lower the solution surface tension to avoid dewetting. Dewetting is defined as spontaneous beading up of liquid on the surface after initial spreading. Dewetting is a highly undesirable event during formation of the membrane sorber, since it leads to non-uniform coating and exposure of the substrate, which sometimes results in non-wettable product and reduced sorptive capacity. The amount of surfactant needed can be conveniently determined by measuring contact angles that a drop of solution makes with a flat surface made from the same material as the porous skeleton. Dynamic advancing and receding contact angles are especially informative, which are measured as the liquid is added to or withdrawn from the drop of solution, respectively. Dewetting can be avoided if the solution is formulated to have the receding contact angle of 0°.

A small amount of a hydrophilic polymer that readily adsorbs on a hydrophobic surface optionally may be added to the solution as a spreading aid. Polyvinyl alcohol is the preferred polymer and can be used in concentrations ranging from about 0.05 wt.% to about 5 wt.% of total solution volume.

When the supporting porous structure cannot be readily wetted with the solution of polymer, such as in the case of hydrophobic microporous membrane, a wetting aid can be added to the solution. The wetting aid can be any organic solvent compatible with the coating polymer solution that does not negatively affect the cross-linking reaction. Typically the solvent is one of the lower aliphatic alcohols, but acetone, tetrahydrofuran, acetonitrile and other water-miscible solvents can be used as well. The amount of the added organic solvent is the minimum needed to effect instant wettability of the porous substrate with the coating solution. Exemplary wetting aids include methyl alcohol, ethyl alcohol, and isopropyl alcohol.

Methods of coating can improve the wetting of the web by the coating solution. The coating solution may be forced into the web in a controlled manner so as to uniformly saturate the web and leave no hydrophobic spots or areas. This may be done, for example by extruding the solution through a slot pressed against the web, or in close proximity to the web, in order to force the solution by the applied pressure of extrusion into the web. Persons skilled in the art will be able to determine conditions of pressure, speed and slot geometry needed to produce a uniform coating.

A preferred process for forming the coated substrate comprises the steps of: 1) preparing the solution; 2) applying the solution on the membrane; removing excess liquid from the external surfaces of the substrate 3) drying the membrane; 4) curing the membrane; 5) rinsing and drying of the membrane; 6) optional annealing of the finished membrane; and 7) optional acid treatment of the membrane. More specifically, a solution is prepared that contains a suitable polymer and cross-linker. The concentrations of these two components determine the thickness and degree of swelling of the deposited coating, which in turn define flux through the membrane and its sorptive capacity. The polymer and cross-linker are dissolved in a suitable solvent, preferably water. The solution may optionally contain other ingredients, such as wetting aids, spreading aids, and a pH adjuster. If a hydrophobic substrate is used, the surface tension of solution will have to be low enough in order to wet it. Aqueous solutions of polymers typically will not wet hydrophobic microporous membranes, so an organic solvent (wetting aid) will have to be added to the solution. To help spread the coating uniformly on the surface of a hydrophobic membrane, a surfactant may be added to the solution. Finally, depending on the chemical nature of the cross-linker, the pH may need to be raised in order to effect the cross-linking reaction. The solution components and typical concentration ranges are listed in Table 1:

**TABLE 1**

| Component | Role | Range, wt.% |
|---|---|---|
| Polymeric primary amine | Hydrogel-forming adsorptive polymer | 3-15 |
| Cross-linker | Effects formation of hydrogel | 0.01-2.0 |
| Surfactant | Surfactant for even coating | 0-3.0 |
| Hydrophilic polymer | Surface hydrophilization | 0-1.0% |
| Organic solvent | Initial wetting of hydrophobic membrane with coating solution | 0-30 |
| Inorganic base | Raise pH to effect cross-linking | 0-5.0 |
| Water | Solvent | Balance |

The coating solution is applied on the substrate such as by submerging the substrate into solution, removing the substrate from solution, and removing excess of solution from both sides of the substrate mechanically, for example, using a pair of nip rolls (Nipped off). The porous substrate whose pores are filled with solution is subsequently dried.

Drying can be carried out by evaporation at room temperature or can be accelerated by applying heat (temperature range of about 40-110°C). After the coated substrate is dried, it is held for a period of from several hours to several days so that cross-linker can fully react with the polymer. Cross-linking may be optionally accelerated by applying heat. The substrate is subsequently rinsed with copious amounts of solvent and dried again. Additional optional process steps include annealing the dried membrane sorber at an elevated temperature (60-120°C) to adjust its flow properties and treating it with a strong non-oxidizing monobasic acid at concentration 0.1M to 1M to protonate the amino groups present in the coating.

Where the polymer is PAA, converting essentially all amino groups in the polymer into corresponding ammonium salts after heat treatment of the membrane will help ensure consistency of the product. Good water wettability is important. Since the base material is very hydrophobic and the hydrophilic coating is very loosely cross-linked and not covalently attached to the matrix, some lateral shrinking of the PAA gel will cause the membrane to become not wettable with water. On the other hand, if essentially all amino groups of the PAA are converted into corresponding ammonium salts, the increased volume of the dried coating, greater retention of water by counter-ions and stronger affinity for water of the charged polymer will help to make the membrane more water-wettable. A strong, non-toxic, non-oxidizing acid, preferably one that is monobasic to avoid ionic cross-linking of PAA, should be used to protonate PAA for this purpose. Suitable acids include hydrochloric, hydrobromic, sulfamic, methansulfonic, trichloroacetic, and trifluoroacetic acid. Although chloride may be the counter-ion of choice since it is already present in the sample protein solution, it may not be practical for a continuous process to use hydrochloric acid and/or its salt due to the corrosion of steel and the occupational safety issues involved. A more suitable acid is thus sulfamic acid ($H_2N-SO_2OH$) is preferred as the protonating agent for PAA.

A suitable process for protonating the PAA is to submerge the membrane in a 0.1-0.5 M solution of the protonating acid, preferably sulfamic acid in water (or a water/alcohol mix to fully penetrate a poorly wetting membrane), followed by rinsing and drying. The resulting membrane will bear sulfamate counter-ions, which may be easily exchanged out by employing a simple conditioning protocol, such as 0.5M sodium hydroxide followed by 0.5M sodium chloride.

Such acid treatment improves shelf life stability of the membrane, and also results in a significantly higher strength of binding. Although the present inventors should not be limited to any particular theory, it is believed that when PAA is dried in the fully protonated (acid-treated) state, it assumes a more extended, "open" morphology that is capable of better encapsulating BSA and thus will not release it until a higher ionic strength is reached. A further benefit of acid-treated membranes is greater stability towards ionizing irradiation, such as gamma irradiation, which is an accepted sterilization procedure for filtration products.

Three critical parameters define a successful membrane sorber product. They are: sorptive capacity, flux, and strength of binding. While the strength of binding is to a large extent determined by the chemical nature of groups presented on the surface of membrane sorber, capacity and flux are usually a lot more sensitive to the procedure employed to form the sorptive layer and the amount of polymer and cross-linker. It is often observed that for a given combination of supporting skeleton, purification efficiency (determined by the bed height) and chemical nature of the membrane sorber, greater flux can translate into smaller sorptive capacity, and vice versa.

The permeability of the cross-linked PAA membrane adsorber was improved by a high-temperature "curing" process. The lightly cross-linked PAA-gel has the ability to absorb significant amount water resulting in orders of magnitude increase in its volume. This effect can cause low permeability. It appears that this property of the gel is reduced by dehydrating it to such an extent that it reduces the swelling to an acceptable level, without compromising the strength of binding and capacity of the gel. In fact, the curing process is capable of tuning the permeability of the membrane as necessary for the product. Suitable curing temperatures are about 25-120°C, more preferably from about 85-100°C; and for about 6 to 72 hours.

Gamma irradiation is a widely accepted sterilization procedure for filtration products. Gamma-sterilizability is a desirable feature of a membrane sorber. The inventors observed a surprising benefit of acid-treated membrane sorber in the fact that it had a greater stability towards ionizing irradiation.

In operation, in accordance with certain embodiments, feed containing the virus of interest as well as impurities such as DNA is introduced into a membrane adsorber in the presence of an ionic-modifier and monovalent salt. Suitable ionic-modifiers include phosphate buffer (e.g., sodium or potassium phosphate), citrate buffer, ethylenediaminetetraacetic acid (EDTA), succinate, ammonium sulfate, sodium sulfate, piperazine-N,N'-bis(2-ethanesulfonic acid), glutamate, tri-polyphosphates, polyelectrolytes such as polyacrylic acid, sodium styrene sulfonate, and surfactants such as sodium lauryl ether sulfate. Suitable monovalent salts include sodium or potassium chloride, sodium bromide, sodium iodide, imidazole hydrochloride, and the like. Suitable ionic modifier concentrations include from about 2 mM to about 0.5M. Suitable monovalent salt concentrations include from about 0 to about 2M.

The following examples are included herein for the purpose of illustration and are not intended to limit the invention.

## Example 1 (PAA on hydrophobic UPE)

Preparation of Polyamine coated membrane

A 20% aqueous solution of isopropyl alcohol containing 9% polyallylamine (PAA, Mw-15000gm/mol), 0.4 % PEGDGE (polyethylene glycol diglycidylether, Mw: 526 gm/mol), 4% lithium hydroxide, 2% TritonX-100, 8% of polyvinyl alcohol solution (2.5% solution) was first prepared. This was coated on a roll of hydrophobic UPE (ultrahigh density polyethylene) membrane with 0.65 $\mu$m pore size using a pilot scale coating machine. After coating, the membrane was extracted with water and methanol to remove excess reactants and dried on a hot air impingement dryer at 120°C. Following this, the membrane were subjected to curing at 95°C for 15 hrs, treated with 5% sulfamic acid in 5% isopropyl alcohol and then dried to stabilize the coating. The membrane so prepared was cut into half inch or one inch discs, stacked into 8 layers, over-molded into polyethylene devices with an inlet and effluent port and further used for flow-through separation studies.

## Example 2

Flow through purification of BSA (model for virus) from DNA using ChromaSorb and Tris Buffer

In this exemplary experiment the flow-through recovery of bovine serum albumin (BSA) and herring sperm DNA using Tris buffer with different concentrations of sodium chloride salt as the mobile phase and a device built as described in Example 1 with the primary amine anion exchanger, is described. The membrane adsorber was built by over-molding 8 layers of membrane (1 inch in diameter, total membrane volume = ~0.34 ml) from Example 1 into polyethylene devices with an inlet and effluent port. BSA was used as a model for proteins as well as viruses that need to be separated from DNA or other nucleotides in a sample. BSA serves as a good model for viruses too because it has a pI of ~5 which is close to the pI of these biomolecules. Herring sperm DNA was used as a model for the host cell DNA and other similar nucleotides that would be found in a typical feed.

Prior to testing, the ChromaSorb devices were prepared by wetting them with 10 ml of water using a syringe. The device was then immersed in a beaker of water with the effluent side facing downwards and with the syringe attached to it. Slight vacuum was then pulled through the device using the syringe to remove any entrapped bubbles. The device was then connected to a pump and flushed with 15 ml of 0.5N NaOH, washed with 15 ml DI water and then pre-equilibrated with 20 ml of 25mM Tris buffer at the respective salt concentrations.

BSA obtained from Sigma Aldrich Corp. was dissolved in three different solutions of Tris buffer (25mM, pH 8) containing 0.5M, 0.7M and 0.9M NaCl respectively to achieve final solutions with a BSA concentration of 1.8 mg/ml. Similarly, herring sperm DNA obtained from Promega Corp. was dissolved in Tris buffers with above mentioned salt concentrations to achieve a final solution with a concentration of 50 $\mu$g/ml. Approximately, 10 ml of each of the BSA containing solutions and 30ml of each of the DNA containing solutions were passed through 6 different ChromaSorb devices (0.33 ml) using a syringe pump (New Era Pump Systems Inc., NE-1600) at a flow rate of 1 ml/min. The concentration of the BSA and DNA in the flow through and feed was measured using a UV-Vis Spectrophotometer (Fisher Scientific) at 280 nm and 260 nm respectively.

The results of an exemplary experiment measuring the recovery of BSA and DNA passed through ChromaSorb devices using Tris buffer are shown in Table 2 below:

**Table 2:**

| Feed Condition | BSA Capacity (mg/ml) | DNA Capacity (mg/ml) | % BSA Recovery | % DNA Recovery |
|---|---|---|---|---|
| 0.5M NaCl 25mM Tris, pH 8 | 28 | > 4.5 | 48 | 0 |
| 0.7M NaCl 25mM Tris, pH 8 | 16 | > 4.5 | 70 | 0 |
| 0.9M NaCl 25mM Tris, pH 8 | 17 | > 4.5 | 67 | 0 |

It can be seen from Table 2 that complete removal of DNA can be achieved using the media in accordance with embodiments disclosed herein under all the above buffer conditions. However, 100% recovery of BSA, which is a model for the proteins/viruses, was not obtained under any of these conditions. This suggests that, although Tris buffer with increasing monovalent salt concentrations can be used as a method for separating DNA from viruses using such media, it might not be possible to achieve complete recovery of the protein/virus using change in salt concentration alone.

### Example 3

Flow-through purification of BSA from DNA using ChromaSorb membrane and Phosphate Buffer.

In this exemplary experiment the flow-through recovery of bovine serum albumin (BSA) and herring sperm DNA using ChromaSorb device (a 0.08 ml single-use, membrane-based anion exchanger commercially available from Millipore Corporation) as the primary amine anion exchanger and buffer solutions containing multivalent phosphate ions and sodium chloride salt as the mobile phase is described. Similar to Example 2, BSA was used as a model for proteins/viruses and herring sperm DNA was used as a model for the host cell DNA.

Prior to testing, the ChromaSorb devices were wetted and degassed as per the method described in Example 2. The devices were then connected to an automated BioCad FPLC system (Applied Biosystems), and were flushed with 0.5N NaOH, washed with water and pre-equilibrated with sodium phosphate buffer (pH 7.1) with or without sodium chloride salt. The FPLC system was further used for measuring the break-through of BSA and DNA from the devices in a flow through mode so as to determine the capacity of the membrane for these biomolecules. After the flow through step the devices were washed with the equilibration buffer, 1M NaCl solution and 0.5N NaOH solution to determine any trace amount of BSA or DNA bound to the membrane.

Separate solutions of BSA (Sigma Aldrich Corp.) and DNA (Promega Corp.) of 0.5mg/ml and 28μg/ml concentrations respectively were prepared in sodium phosphate buffer (pH 7.1) containing different amounts of sodium phosphate or sodium chloride salts. The solutions were passed through two different ChromaSorb devices (0.08 ml) until more than 10% breakthrough of BSA or DNA was recorded at 280 nm and 260 nm respectively on the inline UV-Vis spectrophotometer of the FPLC system. The dynamic capacity of the devices for BSA and DNA at 10% break through was calculated using the following equation:

$$\text{Dynamic capacity} = \frac{C_f \times V_{BT}}{V_d}$$

Where:

$C_f$ = Concentration of the feed;
$V_{BT}$ = Volume of solution that has passed through the device at 10% break through; and
$V_d$ = Volume of the device.

The trace amount of impurities, if any, which were eluted from the device using 1M NaCl solution was used to estimate the device capacity in case the 10% break through curve indicated no measurable capacity. The concentration of BSA eluted was calculated by measuring the area under the elution peak and determining the total amount of protein corresponding to that area against a standard calibration curve and assuming ~65% irreversible binding.

The results of an exemplary experiment measuring the capacity, recovery and removal of BSA and herring sperm DNA using 0.08 ml ChromaSorb devices and sodium phosphate in mobile phase is shown in Table 3.

**Table 3:**

| Concentration of salts in Mobile phase | | BSA Capacity | DNA Capacity | Pool value after ~275CV of feed was processed | |
|---|---|---|---|---|---|
| Sodium Phosphate (mM) | NaCl (M) | (mg/ml) | (mg/ml) | % BSA recovery | % DNA removal |
| 2 | 0 | 58 | NA | 0 | 100 |
| 5 | 0 | 9.7 | NA | 93 | 100 |
| 5 | 0.1 | 4.1 | NA | 97 | 100 |
| 10 | 0 | 1.2 | 7.4 | >99 | 100 |

(continued)

| Concentration of salts in Mobile phase | | BSA Capacity | DNA Capacity | Pool value after ~275CV of feed was processed | |
|---|---|---|---|---|---|
| Sodium Phosphate (mM) | NaCl (M) | (mg/ml) | (mg/ml) | % BSA recovery | % DNA removal |
| 10 | 0.1 | 1.7 | 10 | >99 | 100 |
| 50 | 0 | 0 | 7.3 | 100 | 100 |
| 50 | 0.1 | NA | 10.7 | NA | 100 |
| 50 | 0.3 | 0 | 9.2 | 100 | 100 |
| 50 | 0.5 | NA | 9 | NA | 100 |
| NA- Not available. | | | | | |

As can be seen from Table 3, the BSA capacity of the primary amine membrane reduces drastically with increase in phosphate concentration with little change in DNA capacity. There appears to be a threshold concentration of phosphate ions between 2 mM and 5 mM which causes a drastic increase in protein recovery. The data suggests that complete recovery or extremely high recovery with trace amount of losses protein/virus can be achieved in flow-through mode using phosphate salts at concentrations of 10 mM or more in the mobile phase through a primary amine membrane. The use of sodium chloride salt along with phosphate reduces capacity of the membrane for BSA and improves its binding capacity for DNA. Further, there appears to be an optimum NaCl concentration at which maximum DNA removal can be achieved. The use of NaCl may allow use of the primary membrane at phosphate concentrations as low as 5 mM with very little product loss or as high as 50 mM with enhancement in DNA removal. Thus depending on the application the phosphate and NaCl concentrations can be optimized to allow processing of large volumes of feed with high protein/ virus recoveries and DNA removal. The study suggests that significant DNA removal and complete virus recovery can be achieved in a flow-through mode using a primary amine membrane such as ChromaSorb by using a multivalent salt such as sodium phosphate. Further the use of monovalent salts such as sodium chloride can improve the performance of this separation.

### Example 4

Flow-through purification of Influenza virus form host cell DNA using ChromaSorb membrane and Phosphate Buffer

In another exemplary experiment, the recovery of influenza virus-type A (Flu-A), from a complex feed mixture of host cell protein (HCP) and host cell DNA is described. Influenza-type A is a lipid enveloped virus with a pI of ~5.0 and a size of approximately 80 to 120 nm. The study was performed in flow through mode by flowing clarified and buffer exchanged flu feed through a device containing ChromaSorb (primary amine) membrane.

Feed containing influenza type A virus grown in MDCK cells was prepared using standard procedure. Initially, fifty T150 flasks were seeded at 10% confluency MDCK cells (mandarin darby canine kidney) in 10% FBS (fetal bovine serum) DMEM (Dulbecco's modified eagle's medium). After 2-3 days, once the cells were 80-90% confluent the media in the flasks was changed to DMEM without serum and the cells were infected with influenza type A/WS (H1N1 strain) which was tissue culture adapted to growth in MDCK cells. The flasks were then incubated at 33 °C in 5% $CO_2$ for 3 days until complete CPE (Cytopathic effect) was observed. The supernatant was subsequently centrifuged at 2500 RPM, filtered through a 0.45 $\mu$m membrane filter to remove cell debris and stored at -80°C before further use.

Just prior to the experiment, approximately 40 ml of feed was thawed at 4°C overnight and then buffer exchanged into sodium phosphate buffer (pH 7.1) using a 10 Kilo-Dalton Centricon centrifugal filter device (Millipore Corporation). The phosphate and sodium chloride concentrations in the mobile phase were chosen based on observations made in Example 3. The feed was then passed through a 0.08 ml ChromaSorb device at a flow rate of 1 ml/min using a positive displacement pump (Mighty-Mini, Scientific Systems Inc.). The flow through the columns was collected in 1 ml fractions and assayed in duplicate for flu and host cell DNA content using hemagglutinin (HA) and pico-green assay respectively. The feed influenza virus and host cell DNA titers were 10240 HAU/ml and 1-1.3 $\mu$g/ml respectively.

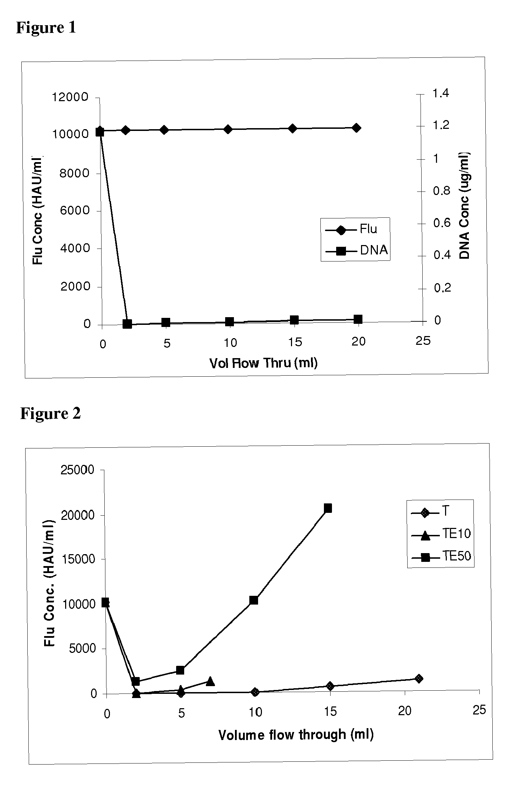

The results of an exemplary experiment measuring the breakthrough of influenza virus type A and host cell DNA passed through a 0.08 ml ChromaSorb device using sodium phosphate buffer as mobile phase are shown in Figure 1.

Table 4: Summary of the capacity and recovery of Influenza type A and host cell DNA obtained using ChromaSorb membrane and phosphate buffer.

| Concentration of salts in Mobile phase | | Flu Capacity | DNA# Capacity | Pool Value after ~187 CV of feed was processed | |
|---|---|---|---|---|---|
| Sodium Phosphate (mM) | NaCl (M) | (kHAU/ml) | (μg/ml) | % Flu recovery | % DNA* removal |
| 50 | 0.1 | 0 | 278 | 100 | 100 |
| 50 | 0.3 | 0 | 239 | 100 | 100 |
| * - DNA concentration bellow 10 ng/ml in the flow-through pool was considered 100% removal.<br># - DNA capacity was calculated at ~10 ng/ml DNA concentration in the flow-though pool. | | | | | |

Figure 1 and Table 4 show that the use of multivalent phosphate salts in the mobile phase allows complete recovery of influenza virus while maintaining significant membrane capacity for host cell DNA. While the capacities for host cell DNA is lower than in the single component system shown in Example 3, the break through of influenza virus and host cell DNA resemble that of BSA and herring sperm DNA in Example 3. This suggests that BSA is a good model for proteins as well as viruses and herring sperm DNA is a good model for host cell DNA. The monovalent salt concentrations can be further optimized to achieve higher DNA removal and product through put. The study confirms that viruses can be separated from host cell DNA in flow-through mode using primary amine media such as ChromaSorb and multivalent ions such as phosphate. Further, presence of monovalent salts such as sodium chloride is conducive for this separation.

## Example 5

Use of phosphate as an additive in Tris buffer to enable flow-through purification of BSA from DNA using ChromaSorb membrane.

In this exemplary experiment the flow-through recovery of BSA and herring sperm DNA using ChromaSorb membrane device (0.08 ml) and Tris buffer as mobile phase is described. Different molar concentrations of sodium phosphate and sodium chloride salts were used as additives to Tris buffer and the final solution was adjusted to pH 8 using 10N NaOH or 2N HCl (Fisher Scientific). This solution was used as the equilibration, load and wash buffer. Salt conditions were chosen based on observations made in Example 3. The experiments were performed in a manner similar to that described in Example 3.

The results of the exemplary experiment measuring the capacity, recovery and removal of BSA and herring sperm DNA, using 0.08 ml ChromaSorb membrane devices and Tris buffer with multivalent phosphate ions as additive is shown in Table 5.

Table 5:

| Concentration of salts in Mobile phase | | | BSA Capacity | DNA Capacity | Pool value after ~275CV of feed was processed | |
|---|---|---|---|---|---|---|
| Tris (mM) | Sodium Phosphate (mM) | NaCl (M) | (mg/ml) | (mg/ml) | % BSA recovery | % DNA removal |
| 50 | 0 | 0 | 57.2 | 23 | 58 | 100 |
| 50 | 0 | 0.1 | NA | 18 | NA | 100 |
| 50 | 50 | 0 | 0.35 | NA | >99 | NA |
| 50 | 50 | 0.3 | 0.19 | 9 | >99 | 100 |

As shown in Table 5, Chromasorb has very high capacity for BSA and DNA in Tris buffer as a result the recovery of the protein is significantly low. Addition of sodium phosphate salt almost eliminates the capacity of the primary amine membrane for BSA allowing greater than 99% recovery of the protein while retaining membrane capacity for herring sperm DNA. As observed earlier addition of sodium chloride salt has the advantage of further improving the protein recovery without affecting DNA removal. The study suggests that in addition to using multivalent salts as buffers they can be simply used as additives to other buffers to purify protein or viruses from DNA in a flow-through mode using primary amine membranes such as ChromaSorb.

## Example 6

Use of phosphate as an additive in Tris buffer to enable flow-through purification of Influenza virus from host cell DNA using ChromaSorb membrane.

In this exemplary experiment the flow-through separation of influenza virus type-A from host cell DNA using ChromaSorb membrane device (0.08 ml) and Tris buffer as mobile phase is described. Sodium phosphate and sodium chloride salts were used as additives to Tris buffer and the pH of the final solution was adjusted to pH 8 using 10N NaOH or 2N HCl. Buffer conditions were chosen based on observations made in Example 5. This solution was used as equilibration as well as load buffer. The virus was propagated and buffer exchanged in a manner similar to that described in Example 4. Further flow-through separation experiments and assays were also performed as described in Example 4. The feed influenza virus and host cell DNA titers were 10240 HAU/ml and 1-1.5 $\mu$g/ml respectively.

**Table 6**: Summary of the capacity and recovery of influenza virus and host cell DNA obtained using ChromaSorb membrane device and Tris buffer containing phosphate ions as mobile phase.

| Concentration of salts in Mobile phase | | | Flu Capacity | DNA[#] Capacity | Pool Value after ~187 CV of feed was processed | |
|---|---|---|---|---|---|---|
| Tris (mM) | Sodium Phosphate (mM) | NaCl (M) | (kHAU/ml ) | ($\mu$g/ml) | % Flu recovery | % DNA* removal |
| 50 | 0 | 0 | > 1700 | 372 | 1.1 | 100 |
| 50 | 50 | 0.3 | 0 | 203 | 100 | 100 |
| * - DNA concentration bellow 10 ng/ml in the flow-through pool was considered 100% removal. [#] - DNA capacity was calculated at ~10 ng/ml DNA concentration in the flow-though pool. | | | | | | |

As observed in Table 6, in Tris buffer Chromasorb membrane had very high capacity for influenza virus which led to extremely poor virus recovery in the flow-through mode. However, addition of sodium phosphate to Tris buffer completely eliminated the capacity of the membrane for the virus allowing complete recovery of the virus while still retaining considerable membrane capacity for host cell DNA. Further optimization of buffer conditions by adjusting phosphate and/or sodium chloride concentrations can be used as an approach to improve the performance of the membrane for this purification. The study supports observations made in Example 5, that a multivalent salt can be used as an additive in an existing buffer to enable flow-through purification of viruses from host cell DNA.

## Example 7

Use of citrate as an additive in Tris buffer to enable flow-through purification of BSA from DNA using ChromaSorb membrane.

In this exemplary experiment the flow-through recovery of BSA and herring sperm DNA using ChromaSorb membrane device (0.08 ml) and Tris buffer as mobile phase is described. Different molar concentrations of citric acid and sodium chloride salts were used as additives to Tris buffer and the final solution was adjusted to pH 8. This solution was used as the equilibration, load and wash buffer. The experiments were performed in a manner similar to that described in Example 3.
The results of the exemplary experiment measuring the capacity, recovery and removal of BSA and herring sperm DNA, using 0.08 ml ChromaSorb membrane devices and Tris buffer with citric acid as additive is shown in Table 7.

**Table 7**:

| Concentration of salts in Mobile phase | | | BSA Capacity | DNA Capacity | Pool value after ~275CV of feed was processed | |
|---|---|---|---|---|---|---|
| Tris (mM) | Citrate (mM) | NaCl (M) | (mg/ml) | (mg/ml) | % BSA recovery | % DNA removal |
| 50 | 0 | 0 | 57.2 | 23 | 58 | 100 |
| 50 | 0 | 0.1 | NA | 18 | NA | 100 |
| 50 | 25 | 0 | 51.2 | NA | 62 | NA |
| 50 | 50 | 0 | 0.24 | NA | >99 | NA |
| 50 | 50 | 0.3 | 0.13 | 17.5 | >99 | 100 |

It can be observed from Table 7 that lower concentration of Citrate ions in Tris buffer did not significantly decrease the membrane capacity for BSA. However, increasing the citrate concentration from 25mM to 50mM had a drastic affect on the membrane capacity and improvement in BSA recovery from 62% to grater than 99%. This suggests that there exists a threshold concentration of citrate ions in Tris buffer that is required for complete recovery of protein in flow-through mode. This threshold concentration may vary for different ionic-modifiers. Further it can be observed that the herring sperm DNA capacity of the membranes in presence of citrate ions is much higher than in phosphate ions. Hence choosing the appropriate ionic-modifier may be an important variable to consider while optimizing buffer conditions for this type of separation.

**Example 8**

Use of citrate as an additive in Tris buffer to enable flow-through purification of Influenza virus from host cell DNA using ChromaSorb membrane.

In this exemplary experiment the flow-through separation of Influenza virus type-A from host cell DNA using ChromaSorb membrane device (0.08 ml) and Tris buffer as mobile phase is described. Citric acid and sodium chloride salts were used as additives to Tris buffer and the pH of the final solution was adjusted to pH 8 using 10N NaOH or 2N HCl. This solution was used as the equilibration as well as load buffer. Conditions were chosen based on observations made in Example 7. The virus was propagated and buffer exchanged in a manner similar to that described in Example 4. Further flow-through separation experiments and assays were also performed as described in Example 4. The feed influenza virus and host cell DNA titers were 10240 HAU/ml and 1-1.5 $\mu$g/ml respectively.

**Table 8**: Summary of the capacity and recovery of Influenza virus and host cell DNA obtained using ChromaSorb membrane device and Tris buffer containing citrate ions as mobile phase.

| Concentration of salts in Mobile phase | | | Flu Capacity | DNA# Capacity | Pool Value after ~187 CV of feed was processed | |
|---|---|---|---|---|---|---|
| Tris (mM) | Citrate (mM) | NaCl (M) | (kHAU/ml ) | ($\mu$g/ml) | % Flu recovery | % DNA* removal |
| 50 | 0 | 0 | > 1700 | 372 | 1.1 | 100 |
| 50 | 50 | 0.3 | 0 | 463 | 100 | 100 |
| * - DNA concentration bellow 10 ng/ml in the flow-through pool was considered 100% removal. # - DNA capacity was calculated at ~10 ng/ml DNA concentration in the flow-though pool. | | | | | | |

Table 8 shows that the use of citrate ions as an additive to Tris buffer has an affect similar to that of phosphate ions in terms of influenza virus recovery. However, the host cell DNA capacity ChromaSorb membrane in presence of citrate ions in Tris buffer is much higher than in presence of phosphate ions. In fact ChromaSorb appeared to have higher capacity in presence of citrate ions than with plain Tris buffer alone. The improvement in DNA capacity over that of Tris buffer is unexpected and not very well understood but suggests that ionic-modifiers analogous to citrate may have a similar affect. Thus citric acid or similar ionic-modifiers by themselves or in combination with monovalent salts such as sodium chloride can be used as additives in existing buffers to achieve highly efficient purification of viruses from host cell DNA using ChromaSorb membrane.

### Example 9

Use of ethylenediaminetetraacetic acid (EDTA) as an additive in Tris buffer to enable flow-through purification of BSA from DNA using ChromaSorb membrane.

In this exemplary experiment the flow-through recovery of BSA and herring sperm DNA using ChromaSorb membrane device (0.08 ml) and Tris buffer as mobile phase is described. Different molar concentrations of ethylenediaminetetraacetic acid (EDTA) and sodium chloride salts were used as additives to Tris buffer and the final solution was adjusted to pH 8. This solution was used as the equilibration, load and wash buffer. The experiments were performed in a manner similar to that described in Example 3.

The results of the exemplary experiment measuring the capacity, recovery and removal of BSA and herring sperm DNA, using 0.08 ml ChromaSorb membrane devices and Tris buffer with EDTA as additive is shown in Table 9.

**Table 9**

| Concentration of salts in Mobile phase | | | BSA Capacity | DNA Capacity | Pool value after ~275CV of feed was processed | |
|---|---|---|---|---|---|---|
| Tris (mM) | EDTA (mM) | NaCl (M) | (mg/ml) | (mg/ml) | % BSA recovery | % DNA removal |
| 50 | 0 | 0 | 57.2 | 23 | 58 | 100 |
| 50 | 0 | 0.1 | NA | 18 | NA | 100 |
| 50 | 1 | 0 | 11.8 | NA | 91 | NA |
| 50 | 10 | 0 | 0 | 13 | 100 | 100 |
| 50 | 10 | 0.1 | 0 | 19 | 100 | 100 |
| 50 | 10 | 0.3 | 0 | 27.7 | 100 | 100 |
| 50 | 50 | 0 | 0 | 22.2 | 100 | 100 |
| 50 | 50 | 0.1 | NA | 25.7 | NA | 100 |
| 50 | 50 | 0.3 | NA | 31.5 | NA | 100 |
| 50 | 50 | 0.5 | 0 | 29.1 | 100 | 100 |
| 50 | 50 | 0.8 | NA | 28.8 | NA | 100 |

Table 9 shows the effect of increasing EDTA concentration in Tris buffer on ChromaSorb's BSA and DNA capacity. EDTA concentrations as low as 1 mM significantly decreases ChromaSorb's BSA capacity allowing high recovery of the protein. On the other hand increase in EDTA concentration initially decreases the DNA capacity of the membrane followed by an increase in capacity. Addition of sodium chloride salt to Tris buffer alone decreases its capacity for DNA. However, in the presence of EDTA the DNA capacity of the membrane increases. The above effect of EDTA in Tris buffer or sodium chloride in the presence of EDTA is unexpected and not very well understood. The study suggest that use of ionic-modifiers such as EDTA cannot only enable complete recovery of proteins and viruses but also improve the capacity of the membranes for DNA which in turn can allow processing of larger volumes of feed. The study suggests that use of ionic-modifiers by themselves or in combinations with monovalent salts are favorable for protein/virus separation from DNA using primary amine membranes such as ChromaSorb.

### Example 10

Use of ethylenediaminetetraacetic acid (EDTA) as an additive in Tris buffer to enable flow-through purification of Influenza virus from host cell DNA using ChromaSorb membrane.

In this exemplary experiment the flow-through separation of Influenza virus type-A from host cell DNA using ChromaSorb membrane device (0.08 ml) and Tris buffer as mobile phase is described. EDTA and sodium chloride salts were used as additives to Tris buffer and the pH of the final solution was adjusted to pH 8. This solution was used as the equilibration as well as load buffer. Conditions were chosen based on observations made in Example 9. The virus was propagated and buffer exchanged in a manner similar to that described in Example 4. Further flow-through separation experiments and assays were also performed as described in Example 4. The feed influenza virus and host cell DNA titers were 10240 HAU/ml and 1-1.5 $\mu$g/ml respectively.

The results of an exemplary experiment measuring the breakthrough of influenza virus type A in a mixture of host cell protein and host cell DNA passed through a 0.08 ml ChromaSorb device using Tris buffer (T), Tris buffer containing 10mM EDTA and 0.3 M NaCl (TE10) and Tris buffer containing 50mM EDTA and 0.3 M NaCl (TE50) as mobile phase are shown in Figure 2.

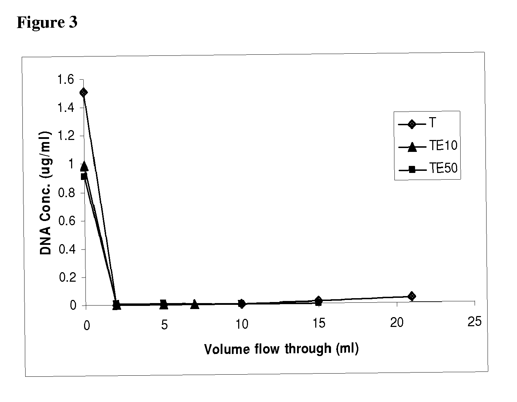

The results of an exemplary experiment measuring the breakthrough of host cell DNA in a mixture of influenza virus type A and host cell protein passed through a 0.08 ml ChromaSorb device using Tris buffer (T), Tris buffer containing 10mM EDTA and 0.3 M NaCl (TE10) and Tris buffer containing 50mM EDTA and 0.3 M NaCl (TE50) as mobile phase is shown in Figure 3.

Table 10: Summary of the capacity and recovery of Influenza virus and host cell DNA obtained using ChromaSorb membrane device and Tris buffer containing EDTA as mobile phase.

| Concentration of salts in Mobile phase | | | Flu Capacity at 88 CV | DNA Capacity at 88 CV | Pool Value after ~88 CV of feed was processed | |
|---|---|---|---|---|---|---|
| Tris (mM) | EDTA (mM) | NaCl (M) | (kHAU/ml) | ($\mu$g/ml) | % Flu recovery | % DNA* removal |
| 50 | 0 | 0 | > 768 | >112.5 | 0 | 100 |
| 50 | 10 | 0.3 | > 742 | > 70.3 | 3.38 | 100 |
| 50 | 50 | 0.3 | > 585 | > 67.4 | 23.7 | 100 |
| * - DNA concentration bellow 10 ng/ml in the flow-through pool was considered 100% removal. | | | | | | |

Due to limited availability of feed only 7 ml of feed containing 10mM EDTA and 15 ml of feed containing 50mM EDTA in Tris buffer were processed through the ChromaSorb devices. As can be seen from Figures 2 and 3 with increase in EDTA concentration from zero to 50mM the break-through of influenza virus occurs much earlier. However, the DNA breakthrough in the presence or absence of EDTA appears to be similar. Thus increase in EDTA concentration results in decrease the virus capacity of the membrane resulting in higher recovery as shown in Table 10. The DNA capacities of the membrane at the two EDTA concentrations are not significantly different. Although the virus recovery does not exactly imitate the behavior of the protein in the presence of EDTA as observed in earlier examples it still follows a same trend of improved virus recovery with increase in ionic-modifier concentration. The difference in the protein and virus capacities with the use of EDTA as an additive may be due to difference in the mechanism of electrostatic shielding by EDTA or due to the differences in the charge strength of the protein, virus and EDTA or the mechanism of interaction of EDTA with the primary amine membrane. Irrespective of the cause of these differences it can be clearly construed from this study that EDTA has inhibitory effects to membrane-virus binding similar to that of other ionic-modifiers discussed earlier. The only difference being higher concentrations of EDTA are required to achieve virus recoveries comparable to that of proteins. Further, use of different buffer conditions such as salts concentration, use of different salt, pH or additives could improve the performance of this separation. Depending on the application these optimizations need to be considered.

## Example 11

Preparation of polyamine grafted Sepharose beads and purification of BSA and influenza virus from DNA using these primary amine modified beads.

In this example the synthesis of Sepharose beads with covalently bound polyallyl amine (PAA) is illustrated. Further, the method of use of these beads for removal of DNA from BSA or influenza virus containing solutions is described. 2g of Epoxy Sepharose 6B (GE Healthcare) beads are suspended in 8 ml of Milli-Q water, to which 10 ml of 10% PAA (Mwt.-3000) solution is added. The pH of the solution is adjusted to 11 using sodium hydroxide and the beads are gently shaken for 24 hrs to enable covalent coupling of the PAA to the bead. Following reaction the beads are rinsed with water and stored wet in the refrigerator before further use. Chromatography columns of 1 ml each are packed with the modified beads and tested for BSA, influenza virus and DNA capacity using ionic modifiers in the mobile phase.

Solutions of 0.5 mg/ml of BSA and 28 $\mu$g/ml herring sperm DNA are prepared separately in 50mM phosphate buffer containing 100mM NaCl. These solutions are separately passed through columns of PAA-Sepharose. The capacity of the PAA-Sepharose beads for BSA and DNA is calculated using the breakthrough curve, as described in Example 3. PAA-Sepharose beads have no or negligible capacity for BSA whereas bind significant amount of DNA. This suggests that proteins can be purified from host cell DNA using polyamine immobilized media such as PAA-Sepharose.

Purification of influenza containing feed is achieved in a manner similar to that described in Example 4. The influenza containing feed is buffer exchanged into 50mM Phosphate buffer containing 100mM NaCl. The feed is passed through the PAA-Sepharose column and the flow through is collected in 1 ml fractions. The fractions are assayed for influenza and DNA content using hemagglutination and Pico-green assay. As observed in Example 4 the influenza virus passes through the column with no or negligible binding to the PAA-Sepharose beads. On the other hand significant amount of host cell DNA bind to the beads reducing the DNA content in the flow through, thereby allowing purification of the virus from host cell DNA in a flow through mode. In a similar manner other ionic modifiers such as such as EDTA or citrate could be used to purify protein and viruses form DNA. Further, the ionic modifiers can be used to make buffers or used as additives in other buffers to achieve this separation using PAA modified beads.

## Claims

1. A method of purifying a sample comprising at least one biomolecule and DNA, said method comprising: purifying said sample by introducing said sample, in the presence of one or more ionic-modifiers optionally in combination with one or more monovalent salts, in an anion exchanger comprising a porous substrate with one or more polymeric primary amines or copolymers thereof formed thereon; and collecting the further purified sample containing said biomolecule free of DNA.

2. The method of claim 1, wherein said one or more ionic-modifiers comprises a buffer.

3. The method of claim 1, wherein said one or more ionic-modifiers comprises phosphate ions.

4. The method of claim 1, wherein said one or more ionic-modifiers comprises citrate ions.

5. The method of claim 1, wherein said one or more ionic-modifier comprises ethylenediaminetetraacetic acid ions.

6. The method of claim 1, wherein said one or more monovalent salts comprises sodium chloride.

7. The method of claim 1, wherein said substrate comprises a microporous membrane.

8. The method of claim 7, wherein said membrane comprises a polyolefin.

9. The method of Claim 8, wherein said polyolefin is polyethylene.

10. The method of claim 1, wherein said substrate is an ultrahigh molecular weight polyethylene membrane.

11. The method of claim 1, wherein said polymer comprises polyallylamine or a protonated polyallylamine.

12. The method of Claim 1, wherein said polymer comprises a copolymer or block copolymer containing polyallylamine or a protonated polyallylamine.

13. The method of claim 1, wherein said polymer is crosslinked.

**Figure 1**

**Figure 2**

**Figure 3**

| | Europäisches Patentamt European Patent Office Office européen des brevets | **EUROPEAN SEARCH REPORT** | **Application Number** EP 11 16 7550 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 027 921 A2 (MILLIPORE CORP [US]) 25 February 2009 (2009-02-25) * the whole document * ----- | 1-13 | INV. C12N7/02 |
| A | EP 2 060 316 A1 (MILLIPORE CORP [US]) 20 May 2009 (2009-05-20) * the whole document * ----- | 1-13 | |
| | | | TECHNICAL FIELDS SEARCHED (IPC) C12N |

The present search report has been drawn up for all claims

| Place of search The Hague | Date of completion of the search 5 October 2011 | Examiner Galli, Ivo |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 16 7550

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2011

| Patent document cited in search report | | Publication date | Patent family member(s) | | Publication date |
|---|---|---|---|---|---|
| EP 2027921 | A2 | 25-02-2009 | EP | 2319622 A2 | 11-05-2011 |
| | | | JP | 2009053191 A | 12-03-2009 |
| | | | SG | 150458 A1 | 30-03-2009 |
| | | | US | 2009050566 A1 | 26-02-2009 |
| | | | US | 2010200507 A1 | 12-08-2010 |
| EP 2060316 | A1 | 20-05-2009 | CN | 101474552 A | 08-07-2009 |
| | | | JP | 2009125071 A | 11-06-2009 |
| | | | SG | 152982 A1 | 29-06-2009 |
| | | | US | 2009130738 A1 | 21-05-2009 |
| | | | US | 2010323430 A1 | 23-12-2010 |

EPO FORM P0459

For more details about this annex : see Official Journal of the European Patent Office, No. 12/82

**EP 2 386 628 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61325954 A
- US 12221496 B